# EUROPÄISCHE PATENTSCHRIFT

(11) **EP 3 197 859 B1**
(45) Veröffentlichungstag und Bekanntmachung des Hinweises auf die Patenterteilung: **06.02.2019**
(21) Anmeldenummer: 15766173.7
(22) Anmeldetag: 21.09.2015
(51) Int. Cl.: C07C 67/08

(54) **VERFAHREN ZUR HERSTELLUNG VON TEREPHTHALSÄUREDIESTERN MIT RÜCKALKOHOL-ENTWÄSSERUNG**
METHOD FOR PRODUCING DIESTERS OF TEREPHTHALIC ACID WITH A DEHYDRATION OF RECIRCULATED ALCOHOL
PROCÉDÉ DE PRODUCTION DE DIESTERS D'ACIDE TÉRÉPHTALIQUE AVEC DÉSHYDRATATION D'ALCOOL RECYCLÉ

(30) Priorität: 24.09.2014 EP 14186143
(43) Veröffentlichungstag der Anmeldung: 02.08.2017
(73) Patentinhaber: BASF SE, 67056 Ludwigshafen (DE)
(72) Erfinder: SCHRAUT, Armin, 64625 Bensheim (DE); KALLER, Martin, 68163 Mannheim (DE); BRONNEBERG, Rob, 67071 Ludwigshafen (DE); STAMMER, Jasmin, 67251 Freinsheim (DE); DAS, Martin, 68163 Mannheim (DE); HARNISCHMACHER, Gerrit, 68159 Mannheim (DE)
(74) Vertreter: BASF IP Association
(86) Internationale Anmeldenummer: PCT/EP2015/071576
(87) Internationale Veröffentlichungsnummer: WO 2016/046118

(56) Entgegenhaltungen:
- EP-A2- 1 186 593
- GB-A- 1 149 460
- GB-A- 1 370 411
- JP-A- 2007 077 041
- US-A- 2 825 738
- US-A- 3 617 226
- US-A- 4 670 580

## Beschreibung

Die Erfindung betrifft ein Verfahren zur Herstellung von Terephthalsäurediestern durch Umsetzung von Terephthalsäure mit wenigstens einem Alkohol.

Ester der Terephthalsäure finden Anwendung als Weichmacher und zeichnen sich durch günstige toxikologische Eigenschaften aus.

Es ist bekannt, Carbonsäureester durch Umsetzung von Carbonsäuren mit Alkoholen herzustellen. Diese Reaktion kann autokatalytisch oder katalytisch, beispielsweise durch Brönstedt- oder Lewissäuren, durchgeführt werden. Unabhängig von der Art der Katalyse entsteht immer ein temperaturabhängiges Gleichgewicht zwischen den Einsatzstoffen (Carbonsäure und Alkohol) und den Produkten (Ester und Wasser).

Um das Gleichgewicht zu Gunsten des Esters (bzw. des Vollesters bei mehrbasigen Säuren) zu verschieben, wird in der Regel ein Schleppmittel eingesetzt, mit dessen Hilfe das Reaktionswasser aus dem Ansatz entfernt wird. Wenn einer der Einsatzstoffe (Alkohol oder Carbonsäure) niedriger siedet als der gebildete Ester und mit Wasser eine Mischungslücke bildet, kann ein Edukt als Schleppmittel verwendet und nach Abtrennung von Wasser wieder in den Ansatz zurückgeführt werden. Bei der Veresterung von höheren aliphatischen Carbonsäuren, aromatischen Carbonsäuren oder von zwei- oder mehrbasigen Carbonsäuren ist in der Regel der eingesetzte Alkohol das Schleppmittel.

Dient der eingesetzte Alkohol als Schleppmittel, geht man üblicherweise so vor, dass man den Brüden aus dem Reaktor zumindest teilweise kondensiert, das Kondensat in eine wässrige Phase und eine im Wesentlichen aus dem zur Veresterung eingesetzten Alkohol bestehende organische Phase trennt und die organische Phase zumindest teilweise in den Reaktor zurückführt.

Die EP-A 1 186 593 beschreibt ein Verfahren zur Herstellung von Carbonsäureestern durch Reaktion von Di- oder Polycarbonsäuren oder deren Anhydriden mit Alkoholen, wobei das Reaktionswasser durch azeotrope Destillation mit dem Alkohol entfernt wird. Die durch die azeotrope Destillation aus der Reaktion entfernte Flüssigkeitsmenge wird vollständig oder teilweise mit dem Alkohol wieder ergänzt.

Die WO 2010/076192 A1 schlägt vor, Leichtsieder aus der zurückzuführenden organischen Phase zu entfernen, um deren Anreicherung im Reaktorsystem zu verhindern.

Die US 7,276,621 B2 beschreibt ein Verfahren zur Titanat-katalysierten Veresterung von Terephthalsäure mit 2-Ethylhexanol. Ein Inertgas wird durch die Reaktionsmischung geleitet, um die Entfernung von Wasser zu unterstützen.

Auch die JP 4956945 B2 beschreibt ein Verfahren zur Veresterung von Terephthalsäure mit 2-Ethylhexanol. Die Terephthalsäure wird dabei als Aufschlämmung kontinuierlich oder diskontinuierlich in das Reaktionssystem eingebracht. Die Zudosierung erfolgt dabei in der gleichen Geschwindigkeit, in der die Terephthalsäure zum Produkt umgesetzt wird.

Die US 7,799,942 B2 beschreibt ein Verfahren zur Herstellung von Terephthalsäurediestern in einem Reaktor bei Atmosphärendruck unter Verwendung einer auf den Reaktor aufgesetzten Destillationskolonne. Zusätzlich wird die Reaktionsmischung von einem Inertgas durchströmt.

Die WO 2010/076193 A1 beschreibt ein Verfahren zur Aufreinigung des rohen Esterproduktes einer Veresterungsreaktion, bei der ein metallhaltiger Veresterungskatalysator eingesetzt wird.

US 2,825,738 A offenbart die Veresterung einer Suspension von Terephthalsäure mit Methanol, in welchem die Mischung von Methanol und Wasser abdestilliert und getrennt wird. Der so erhaltene wasserfreies Methanol wird in das Reaktionssystem zurückgeführt.

Die Löslichkeit von Terephthalsäure in höheren Alkoholen ist gering. Beispielsweise ist Terephthalsäure in 2-Ethylhexanol bei 180 °C nur zu weniger als 0,65 Gew.-% löslich. Die Umsetzung von Terephthalsäure mit höheren Alkoholen verläuft über den Teil an Terephthalsäure, der im Alkohol gelöst vorliegt. Für das Erreichen hoher Umsätze ist es unerlässlich, eine konstante Durchmischung des heterogenen Gemisches von Terephthalsäure und Alkohol und einen effektiven Wärmeeintrag in das Reaktionssystem zu gewährleisten. Weiterhin ist es wichtig den Wassergehalt in der Reaktionsmischung gering zu halten, um das Reaktionsgleichgewicht auf die Produktseite verschieben zu können und, falls hydrolyseempfindliche Veresterungskatalysatoren eingesetzt werden, die Hydrolyse des Katalysators zu verhindern. Die Zudosierung fester Terephthalsäure in den Reaktor mit siedendem Alkohol, z.B. über eine Förderschnecke, bei der das Pulver am freien Ende der Schnecke in freiem Fall in den Reaktor fällt, ist wegen der Gefahr des Verklumpens der Terephthalsäure nur mit Schwierigkeiten möglich. Bei hohen Reaktoren großer Volumina ist das Anbringen eines Vorratsbehälters für Terephthalsäure oberhalb des Reaktors oft mit baulichen Schwierigkeiten verbunden.

Der Erfindung liegt daher die Aufgabe zugrunde, ein Verfahren zur Herstellung von Terephthalsäurediestern bereitzustellen, das einen einfachen Eintrag der Terephthalsäure in den Reaktor erlaubt und einen vollständigen Umsatz der Terephthalsäure erreicht. Eine weitere Aufgabe der Erfindung ist es, ein Verfahren bereitzustellen, das in bestehenden Reaktoren für Veresterungsreaktionen durch geringfügige Umrüstungen durchgeführt werden kann.

Gegenstand der vorliegenden Erfindung ist daher ein Verfahren zur Herstellung eines Terephthalsäurediesters durch Umsetzung von Terephthalsäure mit wenigstens einem Alkohol, wobei man
a) Terephthalsäure in einem Dispergierkessel im Alkohol suspendiert, wobei man eine Vorsuspension erhält,
b) die Vorsuspension aus dem Dispergierkessel in einen Reaktor leitet und in Gegenwart eines Veresterungskatalysators umsetzt, und
c) Reaktionswasser als Alkohol-Wasser-Azeotrop mit dem Brüden abdestilliert, den Brüden zumindest teilweise kondensiert, das Kondensat in eine wässrige Phase und eine organische Phase trennt, die organische Phase entwässert und die entwässerte organische Phase zumindest teilweise in den Dispergierkessel leitet,
und der Alkohol unter linearen, verzweigten oder cyclischen C₈ bis C₁₄ aliphatischen Monoolen, oder aromatischen Monoolen ausgewählt ist.

Das erfindungsgemäße Verfahren kann diskontinuierlich oder kontinuierlich durchgeführt werden, wird aber vorzugsweise diskontinuierlich durchgeführt.

Das Verfahren umgeht die Probleme, die mit dem Zudosieren fester Terephthalsäure in den Reaktor verbunden sind, wie ein Verklumpen der Terephthalsäure und Verstopfen der Förderschnecke oder eines anderen Förderorgans. Das Verfahren sieht die Herstellung einer Vorsuspension in einem Dispergierkessel vor. Terephthalsäure wird nicht als Feststoff sondern in Form einer Suspension in den Reaktor dosiert.

Zur Herstellung der Vorsuspension wird pulverförmige Terephthalsäure im Dispergierkessel in einer Teilmenge des Alkohols suspendiert. Hierzu wird eine geeignete Mischvorrichtung verwendet. So kann eine Menge der Terephthalsäure mit einem Rührwerk mit Alkohol vermischt werden, alternativ können Dispergierpumpen eingesetzt werden. Dabei kann beispielsweise die gesamte Menge an Terephthalsäure in einem Schritt suspendiert werden oder im Verlauf des Verfahrens die Terephthalsäure portionsweise suspendiert werden. Für die portionsweise Suspendierung kann Terephthalsäure z.B. mit Hilfe einer Förderschnecke in den Dispergierkessel dosiert werden.

Die Vermischung kann aber auch in einer geschlossenen Kammer durch die Zusammenwirkung eines sich drehenden Rotors und eines Stators erfolgen, wobei kontinuierlich jeweils nur eine inkrementelle Menge der Komponenten miteinander vermischt wird, und die Suspension danach aus der Kammer austritt.

Als Alkohol zur Bereitung der Vorsuspension wird zumindest teilweise entwässerter Rückalkohol verwendet, d.h. die organische Phase, die nach Kondensation des Brüden, Phasentrennung des Kondensats und Entwässerung der organischen Phase erhalten wird. Zur initialen Bereitung der Vorsuspension zum Anfahren des Verfahrens kann Frischalkohol verwendet werden.

Der Dispergierkessel besteht meist aus metallischen Werkstoffen, wobei Edelstahl bevorzugt ist. Der Dispergierkessel kann gasseitig mit dem Reaktor verbunden sein.

Die Vorsuspension kann mit einer Pumpe in den Reaktor geleitet werden. Alternativ kann die Vorsuspension auch durch die Gravitation in den Reaktor geleitet werden. Als Pumpen sind prinzipiell alle dem Fachmann bekannten Förderpumpen einsetzbar, die dieser unter Berücksichtigung der Eigenschaften der zu fördernden Vorsuspension als geeignet ansieht. Bevorzugt ist als Förderpumpe eine Kreisel-, Kolben-, Schnecken-, Impeller- oder Schlauchpumpe einsetzbar. Die Zudosierung der Vorsuspension in den Reaktor kann portionsweise oder kontinuierlich erfolgen. Vorzugsweise erfolgt die Zudosierung kontinuierlich. Die Vorsuspension kann prinzipiell an jeder Stelle des Reaktors eindosiert werden, vorzugsweise jedoch wird die Vorsuspension im oberen Bereich des Reaktors zugegeben, insbesondere oberhalb des Flüssigkeitsspiegels im Reaktor. Auf diese Weise kann Rückströmung entgegen der Eindosierrichtung weitestgehend verhindert werden.

Beim Reaktor kann es sich um einen beliebigen Reaktor handeln, der zur Durchführung von chemischen Umsetzungen in flüssiger Phase geeignet ist. Als Reaktoren sind nicht rückvermischte Reaktoren, wie Rohrreaktoren oder mit Einbauten versehene Verweilzeitbehälter, vorzugsweise aber rückvermischte Reaktoren, wie Rührkessel, Schlaufenreaktoren, Strahlschlaufenreaktoren oder Strahldüsenreaktoren geeignet. Gegebenenfalls können auch mehrere Reaktoren in einer mehrstufigen Apparatur zusammengefasst werden. Solche Reaktoren sind zum Beispiel Schlaufenreaktoren mit eingebauten Siebböden, kaskadierte Behälter, Rohrreaktoren mit Zwischeneinspeisung oder Rührkolonnen.

Vorzugsweise wird ein Rührkesselreaktor verwendet. Rührkesselreaktoren bestehen meist aus metallischen Werkstoffen, wobei Edelstahl bevorzugt ist.

Insbesondere bevorzugt ist die Verwendung von bestehenden Reaktionssystemen, die z.B. für die Veresterung von Phthalsäureanhydrid genutzt werden, und durch geringfügige Umrüstung für die Veresterung von Terephthalsäure eingesetzt werden können.

Im Reaktor werden die Vorsuspension und der Veresterungskatalysator in Kontakt gebracht, wobei man eine Reaktionssuspension erhält. In einer Ausführungsform des Verfahrens wird dabei i) die Vorsuspension in den unbefüllten Reaktor geleitet, ii) die Vorsuspension zum Sieden erhitzt und iii) der Veresterungskatalysator zugegeben. Gegebenenfalls kann die Reihenfolge der Schritte ii) und iii) umgekehrt werden.

In einer bevorzugten Ausführungsform des Verfahrens wird jedoch der Veresterungskatalysator in einer Teilmenge Alkohol, z.B. 15-50% der Gesamtmenge des Alkohols, vorzugsweise 25-40%, im Reaktor vorgelegt. Die Katalysator/Alkohol-Mischung kann erst zum Sieden erhitzt werden und danach die Zudosierung der Vorsuspension gestartet werden. Alternativ wird die Vorsuspension zur Katalysator/Alkohol-Mischung gegeben und danach erhitzt. Gegebenenfalls können das Erhitzen der Katalysator/Alkohol-Mischung und die Zudosierung der Vorsuspension parallel durchgeführt werden. Insbesondere bevorzugt ist das Zudosieren des Katalysators in die im Reaktor zum Sieden erhitzte Vorsuspension.

Das Erhitzen des vorgelegten Alkohols, der Vorsuspension und/oder einer Katalysator-/Alkoholmischung im Reaktor kann auf beliebige Weise erfolgen, beispielsweise unter Verwendung eines Heizmediums in einem Doppelmantelgefäß, durch Elektrobeheizung, durch Umpumpen des vorgelegten Gemisches über einen außenliegenden Wärmetauscher oder durch ein innenliegendes Heizregister, das unter Verwendung eines Heizmediums, wie Dampf oder Öl, beheizt wird.

Auf gleiche Weise kann der Wärmeeintrag in das Reaktionssystem erfolgen. Vorzugsweise erwärmt man die Reaktionssuspension, indem man sie über einen Wärmetauscher umpumpt, der außerhalb des Reaktors gelegen ist. Als Wärmetauscher kommen prinzipiell alle bekannten Wärmetauscher, beispielsweise Platten- oder Rohrbündelwärmetauscher, oder Kombinationen davon in Betracht.

Zweckmäßigerweise wird die Reaktionssuspension dabei unter Verwendung einer Pumpe aus dem Reaktor abgezogen und durch den Wärmetauscher geleitet. Der Wärmetauscher ist für die Rückführung der erwärmten Reaktionssuspension in den Reaktor fluidleitend mit dem Reaktor verbunden. Die Reaktionssuspension kann prinzipiell an verschiedenen Positionen des Reaktors unterhalb des Flüssigkeitsniveaus der Reaktionssuspension abgezogen werden, vorzugsweise jedoch wird die Reaktionssuspension an der tiefsten Stelle des Reaktors abgezogen. In diesem Fall ist der Reaktor dabei so ausgestaltet, dass die Reaktionssuspension an der geodätisch tiefsten Stelle des Reaktors abgezogen wird und keine Toträume, bedingt durch lokal tiefste Stellen, im Reaktor vorliegen. Die Pumpe zum Abziehen der Reaktionssuspension kann prinzipiell an verschiedenen Positionen außerhalb des Reaktors angeordnet sein. Beispielsweise ist die Pumpe an der geodätisch tiefsten Stelle des aus Reaktor, Pumpe und Verbindungsleitungen bestehenden Kreislaufs angeordnet.

Als Pumpe sind prinzipiell alle dem Fachmann bekannten Förderpumpen geeignet, die dieser zur Durchführung des erfindungsgemäßen Verfahrens unter Berücksichtigung der Eigenschaften der zu fördernden Reaktionssuspension als geeignet ansieht, einsetzbar. Bevorzugt ist als Förderpumpe eine Kreisel-, Kolben-, Schnecken-, Impeller- oder Schlauchpumpe einsetzbar. Ganz besonders bevorzugt wird eine Axial- oder Radial-Kreiselpumpe.

Die Rückführung der Reaktionssuspension in den Reaktor kann prinzipiell an jeder Position des Reaktors erfolgen, zweckmäßigerweise jedoch erfolgt die Rückführung im oberen Bereich des Reaktors, beispielsweise auf Höhe des Flüssigkeitsspiegels der Reaktionssuspension oder im Bereich von der Höhe des Flüssigkeitsspiegels der Reaktorsuspension bis 30% darunter. Der Volumenstrom, der durch die Heizvorrichtung geführt wird, ist beispielsweise so gewählt, dass eine Umwälzung des kompletten Reaktorinhalts in einem Zeitraum von 1 bis 60 Minuten, vorzugsweise 1 bis 10 Minuten erfolgt. Durch die konstante Umwälzung des Reaktorinhalts ist eine effektive Durchmischung der Reaktionssuspension gewährleistet.

Das Umwälzen der Reaktionssuspension kann durch das Eindosieren eines Inertgases in den Reaktor, insbesondere an der tiefsten Stelle des Reaktors, und/oder einen Strom der Reaktionssuspension unterstützt werden. Die Eindosierung des Inertgases trägt insbesondere bei Betriebsstörungen der Pumpe für des Abziehen der Reaktionssuspension, z.B. bei einem Ausfall der Pumpe, dazu bei, eine Sedimentation von Terephthalsäure am Reaktorboden oder in Rohrleitungen zu verhindern. Vorzugsweise erfolgt das Eindosieren des Inertgases an der Saugseite der Pumpe. Alternativ kann die Eindosierung an der Druckseite der Pumpe erfolgen. Inertgase sind alle Gase, die unter den Reaktionsbedingungen keine Reaktivität mit den Bestandteilen der Reaktionssuspension aufweisen, insbesondere Stickstoff oder Argon. Vorzugsweise dosiert man das Inertgas in einer Menge von 0,01 bis 5 Volumeneinheiten des Inertgases pro Volumeneinheit der Reaktionssuspension pro Stunde ein.

Alternativ oder zusätzlich kann die Durchmischung durch die Verwendung eines Rührers unterstützt werden.

Während der Umsetzung weist die Reaktionssuspension im Reaktor eine Temperatur nahe des Siedepunkts des Reaktionsgemisches auf, beispielsweise eine Temperatur von 150 °C bis 250 °C, vorzugsweise von 185 °C bis 220 °C. Der Siedepunkt der Reaktionssuspension ist abhängig vom Verhältnis von Terephthalsäurediester zu Alkohol und steigt im Verlauf der Reaktion an. Siedender Alkohol in der Reaktionssuspension bedingt Dichteunterschiede, aufgrund derer die Reaktionssuspension im Reaktor zirkuliert und umgewälzt wird.

Während der Reaktion wird ein Alkohol-Wasser-Azeotrop mit dem Brüden abdestilliert, der Brüden zumindest teilweise kondensiert, das Kondensat in eine wässrige Phase und eine organische Phase getrennt.

Zur Kondensation bzw. partiellen Kondensation des Brüden können alle geeigneten Kondensatoren verwendet werden. Diese können mit beliebigen Kühlmedien gekühlt werden. Kondensatoren mit Luftkühlung und/oder Wasserkühlung sind bevorzugt, wobei die Luftkühlung besonders bevorzugt ist.

Das erhaltene Kondensat wird einer Phasentrennung in eine wässrige Phase und eine organische Phase unterzogen. Üblicherweise wird das Kondensat hierzu in einen Phasenscheider (Dekanter) geleitet, wo es durch mechanisches Absetzen in zwei Phasen zerfällt, die getrennt abgezogen werden können. Die wässrige Phase wird abgetrennt und kann, gegebenenfalls nach Aufarbeitung, verworfen oder als Strippwasser bei der Nachbehandlung des Esters verwendet werden.

Die abgetrennte organische Phase enthält entsprechend der thermodynamischen Löslichkeit von Wasser im Alkohol noch gelöstes Wasser und wird vor ihrer weiteren Verwendung entwässert.

Die Entwässerung der organischen Phase kann auf verschiedene Weisen erfolgen, beispielsweise durch Pervaporation unter Verwendung von Membranen (JP-A-04308543), azeotrope Destillation in Gegenwart eines Schleppmittels, wie beispielsweise Cyclohexan, die Behandlung mit Molekularsieben oder Zeolithen (EP-A-205582, GB-A-2151501, EP-A-142157, EP-A-158754, US-A-4407662, US-A-4372857, GB-A-2088739 und FR-A-2719039) oder die Verwendung einer Kombination von Extraktion mit flüssigem Kohlendioxid und einem Molekularsieb und dann fraktionierte Destillation (EP-A-233692).

Molekularsiebe, die geeignet sind, gelöstes Wasser aus einem Alkohol zu adsorbieren, sind gut bekannt. Typischerweise sind solche Molekularsiebe kristallin. Das Molekularsieb ist geeigneterweise ein Zeolith-Molekularsieb mit einem mittleren Porendurchmesser von etwa 3 Ångström. Typische Beispiele für solche Molekularsiebe sind die Zeolithe Typ A, insbesondere 3A, 4A und 5A.

Vorzugsweise jedoch umfasst die Entwässerung der organischen Phase ein Strippen mit Alkoholdampf. Hierzu wird die organische Phase, gegebenenfalls nach Alkohol-Anreicherung, in den oberen Bereich einer Abtriebskolonne geleitet und mit Alkoholdampf im Gegenstrom behandelt. Geeignet als Abtriebskolonne sind insbesondere Bodenkolonnen, Packungskolonnen oder Füllkörperkolonnen. Geeignet sind z. B. Kolonnen mit 2 bis 10 theoretischen Trennstufen, vorzugsweise 3 bis 6 theoretischen Trennstufen. Der Alkoholdampf wird geeigneterweise durch einen Aufheizer erzeugt, der mit dem Sumpf der Abtriebskolonne fluidleitend verbunden ist. Am Kopf der Abtriebskolonne wird ein gasförmiges Wasser/Alkohol-Gemisch abgezogen. Man kann das gasförmige Wasser/Alkohol-Gemisch zumindest teilweise kondensieren, das Kondensat in eine wässrige Phase und eine organische Phase trennen, und die organische Phase zumindest teilweise als Rücklauf in die Abtriebskolonne und/oder in den Reaktor leiten.

Vorzugsweise führt man das gasförmige Wasser/Alkohol-Gemisch vom Kopf der Abtriebskolonne und den Reaktionsbrüden einer gemeinsamen Aufarbeitung zu, wobei man einen gemeinsamen Kondensator und Phasenscheider verwendet.

In einer bevorzugten Ausführungsform führt man in einer Verstärkungskolonne der organische Phase den Brüden aus dem Reaktor entgegen, um eine Alkohol-angereicherte organische Phase zu erhalten, und strippt die Alkohol-angereicherte organische Phase in einer Abtriebskolonne mit Alkoholdampf, um die entwässerte organische Phase zu erhalten. Der Alkoholdampf wird geeigneterweise durch einen Aufheizer erzeugt, der mit dem Sumpf der Abtriebskolonne fluidleitend verbunden ist. Das gasförmige Wasser/Alkohol-Gemisch aus der Abtriebskolonne wird geeigneterweise in den unteren Bereich der Verstärkungskolonne geleitet. Am Kopf der Verstärkungskolonne wird ein gasförmiges Wasser/Alkohol-Gemisch abgezogen. Man kondensiert das gasförmige Wasser/Alkohol-Gemisch zumindest teilweise, trennt das Kondensat in eine wässrige Phase und eine organische Phase, und leitet die organische Phase zumindest teilweise als Rücklauf in die Versärkungskolonne und/oder die Abtriebskolonne. Die Ausführungsform erlaubt eine optimale Wärmeintegration des Verfahrens. Zum Betrieb des Aufheizers der Abtriebskolonne ist Dampf einer niedrigeren Dampfstufe ausreichend als zum Aufheizen der Reaktionssuspension.

Die Verstärkungskolonne ist vorzugsweise direkt auf dem Reaktor angeordnet. Im unteren Bereich der Verstärkungskolonne wird eine Alkohol-angereicherte organische Phase gesammelt. Hierzu ist im unteren Bereich der Kolonne geeigneterweise ein Sammelboden angebracht, z. B. ein Kaminboden mit gleichverteilten bedachten Kaminen. Der Sammelboden weist z.B. ein Gefälle nach Innen und eine zentrale Abzugstasse und Abzugsstutzen auf. Vom Sammelboden der Kolonne wird die gesammelte Alkohol-angereicherte organische Phase in die Abtriebskolonne geleitet. Das Ableiten des Rückalkohols kann durch die Gravitation oder unter Verwendung einer Pumpe erfolgen, vorzugsweise durch Gravitation.

Die Abtriebskolonne ist vorzugsweise außerhalb des Reaktors gelegen und mit dem Abzug der Versärkungskolonne fluidleitend verbunden. Alternativ können Abtriebskolonne und Verstärkungskolonne auch als einstückge Rektifikationskolonne vorliegen, wobei der Reaktionsbrüden in den mittleren Bereich der Kolonne seitlich eingespeist wird.

Durch die Entwässerung wird die organische Phase von Wasserspuren befreit, die nach der Phasentrennung in der organischen Phase verblieben sind bzw. gemäß ihrer thermodynamischen Löslichkeit in der organischen Phase gelöst sind. Der Wassergehalt in der entwässerten organischen Phase ist in der Regel geringer als die Löslichkeit von Wasser im Alkohol, beträgt aber vorzugsweise weniger als 1000 ppm, insbesondere weniger als 100 ppm. Die Entwässerung der organischen Phase hat den Vorteil, dass der Wassergehalt in der Reaktionsmischung gering gehalten wird, so dass das Reaktionsgleichgewicht auf die Produktseite verschoben werden kann und, falls hydrolyseempfindliche Veresterungskatalysatoren eingesetzt werden, die Hydrolyse des Katalysators verhindert werden kann.

Erfindungsgemäß wird die entwässerte organische Phase zumindest teilweise in den Dispergierkessel geleitet. Dabei kann die entwässerte organische Phase direkt in den Dispergierkessel geleitet werden oder alternativ zunächst in einen beheizten und/oder isolierten Vorratsbehälter geleitet, dort gelagert und von dort in den Dispergierkessel geleitet werden.

Die Vorerwärmung der entwässerten organischen Phase im Vorratsbehälter weist den Vorteil auf, dass Energieverluste durch Abkühlen vermieden werden, zum Aufheizen Dampf niedrigen Druckes verwendet werden kann und die Löslichkeit von Terephthalsäure in warmem Alkohol deutlich größer ist als in kaltem. Vorzugsweise wird die entwässerte organische Phase im Vorratsbehälter auf eine Temperatur unterhalb des Siedepunkts des Alkohols temperiert, insbesondere auf eine Temperatur von 50 °C bis 10°C unterhalb des Siedepunkts des Alkohols. Der Wärmeeintrag in den Vorratsbehälter kann auf beliebige Weise erfolgen, z. B. durch einen außenliegend angeordneten Heizmantel und/oder innenliegende Heizwendeln, die mittels eines Heizmedium auf heizbar sind, oder eine Elektrobeheizung oder durch Umpumpen durch einen externen Wärmetauscher.

Das Ableiten der organischen Phase aus der Rektifikationsvorrichtung, sowie vom Vorratsbehälter in den Dispergierkessel, kann durch die Gravitation oder unter Verwendung einer Fördervorrichtung, z.B. einer Kreisel-, Kolben-, Schnecken-, Impeller- oder Schlauchpumpe, erfolgen.

Die, gegebenenfalls nach Lagerung im beheizten Vorratsbehälter, in den Dispergierkessel geleitete organische Phase steht für die Suspendierung von Terephthalsäure im Dispergierkessel zur Verfügung. Die Dosierung der Terephthalsäure als Vorsuspension kann zeitlich verteilt über die Reaktionsdauer erfolgen. Dadurch entfällt das Erfordernis, konzentrierte Vorsuspensionen handzuhaben. Dies hat den Vorteil, dass die dem Reaktor zugeführte Suspension einen geringen Feststoffgehalt aufweist und somit gut gefördert werden kann. Weiterhin ist durch die Verwendung von Rückalkohol die Feststoffkonzentration im Reaktor ebenfalls gering, wodurch das Reaktionsvolumen optimal genutzt werden kann. Ein weiterer Vorteil ist, dass bei Ausfall der Fördervorrichtung weniger Probleme durch Sedimentation auftreten.

Im erfindungsgemäßen Verfahren werden bevorzugt lineare, verzweigte oder cyclische aliphatische Alkohole mit 8 bis 14 C-Atomen, oder aromatische Alkohole eingesetzt. Die Alkohole sind Monoole und können tertiär, sekundär oder primär sein.

Die eingesetzten Alkohole können aus verschiedenen Quellen stammen. Geeignete Einsatzstoffe sind beispielsweise Fettalkohole, Alkohole aus dem Alfolprozess oder Alkohole oder Alkoholgemische, die durch Hydrierung von gesättigten oder ungesättigten Aldehyden gewonnen wurden, insbesondere solchen, deren Synthese einen Hydroformylierungsschritt einschließt.

Aliphatische Alkohole, die im erfindungsgemäßen Verfahren eingesetzt werden, sind beispielsweise Octanole, wie n-Octanol, 2-Ethylhexanol, Nonanole, Decylalkohole oder Tridecanole hergestellt durch Hydroformylierung oder Aldolkondensation und anschließende Hydrierung. Die Alkohole können als reine Verbindung, als Gemisch isomerer Verbindungen oder als Gemisch von Verbindungen mit unterschiedlicher C-Zahl eingesetzt werden. Ein Beispiel eines derartigen Alkoholgemisches ist ein C₉/C₁₁-Alkoholgemisch.

Aromatische Alkohole, die im erfindungsgemäßen Verfahren eingesetzt werden können, sind beispielsweise Phenol, Benzylalkohol, 1-Naphtol, 2-Naphtol, 2,4,6-Trinitrophenol, primärer Phenylethylalkohol, sekundärer Phenylethylalkohol, Phenylpropylalkohol, o-Tolylalkohol, p-Tolylalkohol, Cuminalkohol, p-Nitrophenol, m-, o- oder p-Alkylphenol, z.B. m-, o- oder p-Methylphenol oder m-, o- oder p-Ethylphenol, m-, o- oder p-Halogenphenol, z.B. m-, o- oder p-Chlorphenol oder m-, o- oder p-Bromphenol. Weiterhin können p-Nitrobenzylalkohol, m-, o- oder p-Alkylbenzylalkohol, z.B. m-, o- oder p-Methylbenzylalkohol oder m-, o- oder p-Ethylbenzylalkohol, m-, o- oder p-Halogenbenzylalkohol, z.B. m-, o- oder p-Chlorbenzylalkohol oder m-, o- oder p-Brombenzylalkohol, 2-Ethoxyphenol, 3-Ethoxyphenol, 4-Ethoxyphenol, 2-Propoxyphenol, 3- Propoxyphenol, 4- Propoxyphenol, 2-Ethoxybenzylalkohol, 3-Ethoxybenzylalkohol, 4-Ethoxybenzylalkohol, 2-Propoxybenzylalkohol, 3-Propoxybenzylalkohol oder 4-Propoxybenzylalkohol eingesetzt werden.

Besonders bevorzugte Alkohohole sind 2-Ethylhexanol, 2-Propylheptanol, Isononanol-Isomerengemische, Decanol-Isomerengemische und C₉/C₁₁-Alkoholgemische.

Der umzusetzende Alkohol, der als Schleppmittel dient, kann im stöchiometrischen Überschuss eingesetzt werden. Vorzugsweise wird die Menge an eingesetztem Alkohol so gewählt, dass im Rohprodukt der Umsetzung 10 bis 35 Gew.-% Alkohol vorliegen, bezogen auf den theoretischen Vollumsatz der Terephthalsäure.

Die erfindungsgemäße Veresterung wird in Gegenwart eines Veresterungskatalysators durchgeführt.

In einer bevorzugten Ausführungsform des erfindungsgemäßen Verfahrens ist der Veresterungskatalysator im Alkohol löslich.

Geeigneterweise ist der Veresterungskatalysator unter Lewissäuren, wie Alkoholaten, Carboxylaten und Chelatverbindungen von Titan, Zirkonium, Hafnium, Zinn, Aluminium und Zink; Bortrifluorid, Bortrifluorid-Etheraten; Mineralsäuren, wie Schwefelsäure, Phosphorsäure; Sulfonsäuren, wie Methansulfonsäure und Toluolsulfonsäure, und ionischen Fluiden ausgewählt.

Geeigneterweise ist der Veresterungskatalysator unter Alkoholaten, Carboxylaten und Chelatverbindungen von Titan, Zirkonium, Hafnium, Zinn, Aluminium und Zink ausgewählt. Es eignen sich Tetraalkyltitanate, wie Tetramethyltitanat, Tetraethyltitanat, Tetra-n-propyltitanat, Tetra-isopropyltitanat, Tetra-n-butyltitanat, Tetra-isobutyltitanat, Tetrasec-butyltitanat, Tetraoctyltitanat, Tetra-(2-ethylhexyl)-titanat; Dialkyltitanate ((RO)₂TiO, worin R z.B. für iso-Propyl, n-Butyl, iso-Butyl steht), wie Isopropyl-n-butyltitanat; TitanAcetylacetonat-Chelate, wie Di-isopropoxy-bis(acetylacetonat)titanat, Di-isopropoxy-bis(ethylacetylacetonat)titanat, Di-n-butyl-bis(acetylacetonat)titanat, Di-n-butyl-bis(ethylacetoacetat)titanat, Tri-isopropoxid-bis(acetylacetonat)titanat; Zirkontetraalkylate, wie Zirkontetraethylat, Zirkontetrabutylat, Zirkontetrabutyrat, Zirkontetrapropylat, Zirkoncarboxylate, wie Zirkondiacetat; Zirkon-Acetylacetonat-Chelate, wie Zirkontetra(acetylacetonat), Tributoxyzirkonacetylacetonat, Dibutoxyzirkon(bis-acetylacetonat); Aluminiumtrisalkylate, wie Aluminiumtriisopropylat, Aluminiumtrisbutylat; Aluminium-Acetylacetonat-Chelate, wie Aluminiumtris(acetylacetonat) und Aluminiumtris(ethylacetylacetonat). Insbesondere werden Isopropyl-n-butyltitanat, Tetra(isopropyl)orthotitanat, Tetra(butyl)orthotitanat oder Mischungen davon eingesetzt.

Geeignete ionische Fluide (ionic liquids) sind z. B. Methylimidazoliumbutansulfonsäuretriflat und 1-Ethyl-3-methyl-imidazolium-hydrogensulfat.

Die Katalysatorkonzentration hängt von der Art des Katalysators ab. Bei den bevorzugt eingesetzten Titanverbindungen beträgt diese 0,001 bis 1,0 mol% bezogen auf die Menge an Terephthalsäure, insbesondere von 0,01 bis 0,2 mol%.

Die Reaktionstemperaturen liegen in der Regel zwischen 100 °C und 250 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Olefinbildung oder Bildung farbiger Nebenprodukte. Es ist zur Entfernung des Reaktionswassers erforderlich, dass der Alkohol aus dem Reaktionsgemisch abdestillieren kann. Die gewünschte Temperatur oder der gewünschte Temperaturbereich kann durch den Druck im Reaktor eingestellt werden. Bei niedrig siedenden Alkoholen kann daher die Umsetzung bei Überdruck und bei höher siedenden Alkoholen bei vermindertem Druck durchgeführt werden. Beispielsweise wird bei der Umsetzung von Terephthalsäure mit 2-Ethylhexanol in einem Temperaturbereich von 180 °C bis 220 °C im Druckbereich von 300 mbar bis 2 bar gearbeitet.

Zweckmäßigerweise werden Reaktor und Dispergierkessel bei im Wesentlichen gleichem Druck betrieben, insbesondere etwa Umgebungsdruck. Gegebenenfalls können Reaktor und Dispergierkessel auch bei unterschiedlichen Drücken betrieben werden.

Vorzugsweise wird das erfindungsgemäße Verfahren durchgeführt, bis die Terephthalsäure im Wesentlichen vollständig umgesetzt ist. Die Bestimmung des Umsatzes kann über die Bestimmung der Säurezahl der Reaktionssuspension erfolgen. Die Säurezahl wird durch Neutralisation einer Probe der Reaktionssuspension mit Tetrabutylammoniumhydroxyd bestimmt. Über die, bei der Neutralisation verbrauchte, Masse an Tetrabutylammoniumhydroxyd kann die Stoffmenge der verbrauchten Tetrabutylammoniumhydroxyd bestimmt werden und über stöchiometrische Betrachtungen die Stoffmenge an freien Säuregruppen nicht umgesetzter Terephthalsäure. Ausgehend von der bekannten Stoffmenge der eingesetzten Terephthalsäure kann so der Umsatz ermittelt werden. Zusätzliche Möglichkeiten zur Bestimmung des Umsatzes stellen HPLC-Messungen und die Messung der Trübung der Reaktionssuspension durch in-line-Trübungsmessungen dar. Im erfindungsgemäßen Verfahren wird vorzugsweise ein Umsatz größer als 99% erreicht.

Nach Beendigung der Reaktion enthält das Reaktionsgemisch, das im Wesentlichen aus dem gewünschten Ester und überschüssigem Alkohol besteht, neben dem Katalysator und/oder dessen Folgeprodukte geringe Mengen an Estercarbonsäure(n) und/oder nicht umgesetzter Carbonsäure.

Zur Aufarbeitung dieser Esterrohgemische wird der rohe Terephthalsäurediester mit einer wässrigen Base versetzt, aus dem erhaltenen Gemisch Wasser abdampft, die erhaltene flüssige Phase unter Bildung einer Wasser-in-ÖI-Emulsion mit Wasser versetzt, aus der Emulsion Wasser abdestilliert und der Terephthalsäurediester filtriert.

Zuerst wird der Veresterungskatalysator durch Zugabe einer wässrigen Base desaktiviert und ausgefällt. Gleichzeitig werden die bei der Veresterungsreaktion nicht umgesetzte Säure bzw. Partialester der Säure in Salze überführt.

Die Zugabe der wässrigen Base kann auf beliebige geeignete Weise erfolgen. Sie erfolgt vorzugsweise unterhalb der Flüssigkeitsoberfläche des rohen Esters. Hierfür eignen sich z. B. Lanzen oder Düsen, die an einem Behälterboden oder der Behälterwand vorgesehen sind. Das Gemisch wird dann intensiv durchmischt, z. B. mittels Rührer oder einer Umwälzpumpe.

Die zugegebene Menge wässrige Base ist so bemessen, dass sie zur vollständigen Neutralisation der sauren Komponenten des rohen Esters ausreicht. In der Praxis wird ein mehr oder weniger großer Überschuss an Base eingesetzt. Die Gesamtmenge der sauren Komponenten des rohen Esters wird zweckmäßig durch die Säurezahl (in mg KOH/g) erfasst. Vorzugsweise bringt man mit der wässrigen Base 100 bis 300% Neutralisationsäquivalente ein, bezogen auf die Säurezahl des rohen Esters, insbesondere 130 bis 220%. Unter Neutralisationsäquivalent wird dabei die Menge Base verstanden, die die gleiche Zahl Protonen binden kann, wie 1 mg KOH. Mit anderen Worten verwendet man einen Basenüberschuss von bis zu 200%, vorzugsweise 30 bis 120%.

Als wässrige Base kommen Lösungen von Hydroxiden, Carbonaten, Hydrogencarbonaten von Alkalimetallen und Erdalkalimetallen in Betracht. Wässrige Alkalimetallhydroxidlösungen sind im Allgemeinen bevorzugt. Wässrige Natriumhydroxidlösung ist aufgrund ihrer leichten Verfügbarkeit besonders bevorzugt.

Die Konzentration der wässrigen Base ist an sich nicht kritisch, jedoch kann es beim Einsatz konzentrierter Alkalilösungen an der Einleitstelle der Base zur Hydrolyse der Ester kommen. Andererseits soll die Konzentration der wässrigen Base nicht zu niedrig sein, da das mit der wässrigen Base eingebrachte Wasser im nachfolgenden Schritt wieder entfernt werden muss. Daher sind wässrige Basen mäßiger bis geringer Konzentration bevorzugt, z. B. solche einer Konzentration von 0,5 bis 25 Gew.-%, insbesondere 1 bis 10 Gew.-%. Wässrige Natriumhydroxidlösung mit einer Konzentration von 1 bis 5 Gew.-% ist besonders bevorzugt.

Oft liegt der ausgefallene Feststoff, der im Wesentlichen aus Katalysator-Zersetzungsprodukten und Salzen von nicht umgesetzter Säure bzw. Partialestern mehrbasiger Säuren besteht, in fein verteilter, schwer filtrierbarer Form vor. Zweckmäßigerweise werden die feinen Teilchen zu größeren, leicht abtrennbaren Teilchen agglomeriert.

Hierzu versetzt man die flüssige Phase unter Bildung einer Wasser-in-ÖI-Emulsion mit Wasser. Das Wasser wird als disperse Phase in Form feiner Tröpfchen in der flüssigen organischen Phase verteilt. Die feinen Feststoffteilchen wandern an die Grenzfläche zwischen Wassertröpfchen und umgebender organischer Phase. Bei der nachfolgenden Verdampfung des Wassers agglomerieren die feinen Teilchen und bilden grobe, gut abtrennbare Teilchen.

Damit sich eine eigene Wasserphase ausbildet, muss die zugegebene Wassermenge größer sein, als der Löslichkeit von Wasser in der organischen Phase entspricht. Die Wasserlöslichkeit in der organischen Phase hängt unter anderem vom Gehalt an nicht umgesetzten Alkohol ab, da der Alkohol als Lösungsvermittler wirkt. Je höher der Alkoholgehalt, umso mehr Wasser muss zur Emulsionsbildung zugesetzt werden. Bei üblichen Restalkoholgehalten von 20 bis 30 Gew.-% sind im Allgemeinen Mengen von 10 bis 80 g Wasser, vorzugsweise 30 bis 50 g, bezogen auf 1 kg rohen Ester, geeignet.

Die Wasserphase wird mit einem geeigneten Rührer oder Homogenisator oder durch Umpumpen der Emulsion unter Verwendung einer Umwälzpumpe in feine Tröpfchen zerteilt. Die erzeugten Wassertröpfchen weisen vorzugsweise eine mittlere Tröpfchengröße von weniger als 1000 µm auf. Als Rührer mit hoher spezifischer Rührleistung eignen sich beispielsweise Scheibenrührer. Alternativ kann man besonders bei kontinuierlicher Verfahrensführung eine Mischdüse verwenden, bei der über ein Dispergierventil Wasser direkt in den Rohesterstrom zugegeben wird.

Die Emulsionsbildung erfolgt zweckmäßigerweise bei etwa Normaldruck.

Aus der so erzeugten Emulsion wird im nächsten Schritt das Wasser wieder abdestilliert.

Nach dieser Behandlung liegt der Feststoff in gut filtrierbarer Form vor; es schlägt kein Feinanteil bei der Filtration durch. Zur Filtration des Esters eignen sich alle geeigneten Filter wie Kammerfilterpressen, Bandfilter, Kerzenfilter oder Tellerfilter. Zur kontinuierlichen Verfahrensführung eignen sich besonders Tellerfilter mit Zentrifugalkuchenabwurf. Der abgetrennte Feststoff wird verworfen.

Nach der Filtration kann der Ester verschiedenen Nachbehandlungen unterzogen werden, wie einer Dampfstrippung oder dergleichen.

Die Erfindung wird durch die beigefügte Figur näher erläutert.

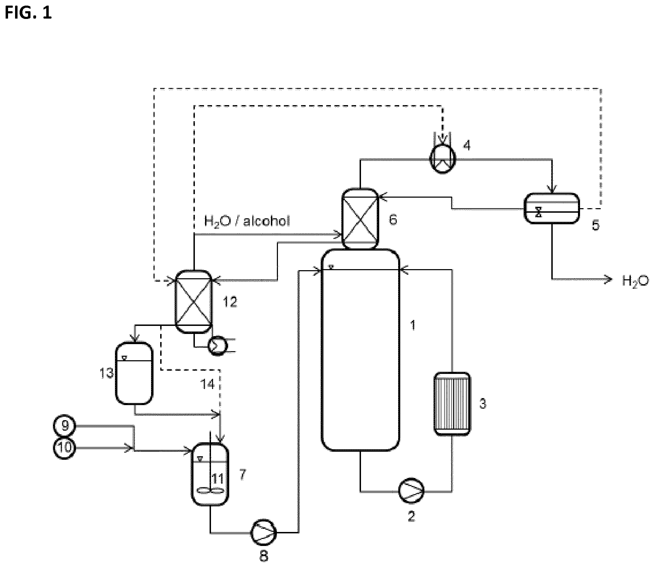

Figur 1 zeigt eine zur Durchführung des erfindungsgemäßen Verfahrens geeignete Anlage.

Gemäß Figur 1 wird in einen Dispergierkessel 7 Terephthalsäure aus dem Reservoir 10 zudosiert und unter Verwendung eines Rührers 11 in Alkohol dispergiert, wobei eine Vorsuspension erhalten wird. Initial wird Alkohol aus dem Reservoir 9 in den Dispergierkessel 7 dosiert. Die Vorsuspension wird mit Hilfe einer Pumpe 8 in den oberen Bereich des Reaktors 1 geleitet. In dem Reaktor 1 befinden sich eine weitere Teilmenge des Alkohols und der Veresterungskatalysator. An der tiefsten Stelle des Reaktors 1 wird die Reaktionssuspension unter Verwendung einer Pumpe 2 aus dem Reaktor abgezogen und durch Wärmetauscher 3 geführt. Die im Wärmetauscher 3 erwärmte Reaktionssuspension wird im oberen Bereich des Reaktors 1 wieder in diesen zurückgeführt. Der Brüden tritt durch die Verstärkungskolonne 6 und wird zumindest teilweise im Kondensator 4 kondensiert. Im Phasenscheider 5 wird das Kondensat in eine wässrige und eine organische Phase getrennt. Die wässrige Phase wird verworfen, die organische Phase wird als Rücklauf in die Verstärkungskolonne 6 geleitet. Alternativ oder zusätzlich kann die organische Phase in die Abtriebskolonne 12 geleitet werden. Im unteren Bereich der Verstärkungskolonne 6 wird eine Alkohol-angereicherte organische Phase gesammelt und in die Abtriebskolonne 12 geleitet. Die Abtriebskolonne 12 umfasst einen Sumpfaufheizer, der die organische Phase teilweise verdampft. Durch die aufsteigenden Alkoholdämpfe werden Wasserspuren aus der Alkoholangereicherten organischen Phase ausgestrippt. Alkohol-Wasser-Dämpfe werden über Kopf abgezogen und in den unteren Bereich der Verstärkerkolonne 6 und/oder den Kondensator 4 geleitet. Sumpfflüssigkeit aus der Abtriebskolonne 12 wird entweder direkt über Leitung 14 in den Dispergierkessel 7 geleitet oder im Vorratsbehälter 13 zwischengelagert.

## Patentansprüche

1. Verfahren zur Herstellung eines Terephthalsäurediesters durch Umsetzung von Terephthalsäure mit wenigstens einem Alkohol, wobei man
a) Terephthalsäure in einem Dispergierkessel im Alkohol suspendiert, wobei man eine Vorsuspension erhält,
b) die Vorsuspension aus dem Dispergierkessel in einen Reaktor leitet und in Gegenwart eines Veresterungskatalysators umsetzt, und
c) Reaktionswasser als Alkohol-Wasser-Azeotrop mit dem Brüden abdestilliert, den Brüden zumindest teilweise kondensiert, das Kondensat in eine wässrige Phase und eine organische Phase trennt, die organische Phase entwässert und die entwässerte organische Phase zumindest teilweise in den Dispergierkessel leitet,
und der Alkohol unter linearen, verzweigten oder cyclischen C₈ bis C₁₄ Monoolen, oder aromatischen Monoolen ausgewählt ist.

2. Verfahren nach Anspruch 1, wobei die entwässerte organische Phase einen Wassergehalt von weniger als 1000 ppm aufweist.

3. Verfahren nach Anspruch 1 oder 2, wobei man die organische Phase durch Strippen mit Alkoholdampf entwässert.

4. Verfahren nach Anspruch 3, wobei man in einer Verstärkungskolonne der organische Phase den Brüden entgegenführt, um eine Alkohol-angereicherte organische Phase zu erhalten, und die Alkohol-angereicherte organische Phase in einer Abtriebskolonne mit Alkoholdampf strippt, um die entwässerte organische Phase zu erhalten.

5. Verfahren nach einem der vorhergehenden Ansprüche, wobei man die entwässerte organische Phase vor dem Einleiten in den Dispergierkessel in einem isolierten und/oder beheizten Behälter lagert.

6. Verfahren nach einem der vorhergehenden Ansprüche, wobei der Veresterungskatalysator unter Lewissäuren, Mineralsäuren, Sulfonsäuren und ionischen Fluiden ausgewählt ist.

7. Verfahren nach Anspruch 7, wobei der Veresterungskatalysator unter Alkoholaten, Carboxylaten und Chelatverbindungen von Titan, Zirconium, Zinn, Hafnium, Aluminium und Zink; Bortrifluorid, Bortrifluorid-Etheraten; Schwefelsäure, Phosphorsäure; Methansulfonsäure und Toluolsulfonsäure ausgewählt ist.

8. Verfahren nach Anspruch 1 bis 7, wobei der Veresterungskatalysator unter sauren Ionentauschern, Zeolithen, Oxiden und/oder Hydroxiden von Magnesium, Aluminium, Zink, Titan, Silicium, Zinn, Blei, Antimon, Bismuth, Molybdän und Mangan ausgewählt ist.

9. Verfahren nach einem der vorhergehenden Ansprüche, wobei der Veresterungskatalysator im Alkohol löslich ist.

10. Verfahren nach einem der vorhergehenden Ansprüche, wobei man den Alkohol in einem solchen stöchiometrischen Überschuss einsetzt, dass das rohe Veresterungsprodukt 15 bis 35 Gew.-% Alkohol enthält.

11. Verfahren nach einem der vorhergehenden Ansprüche, wobei man ein Inertgas zur Fluidisierung in den Reaktor und/oder einen Strom der Reaktionssuspension eindosiert.

12. Verfahren nach einem der vorhergehenden Ansprüche, wobei man zur Aufarbeitung den rohen Terephthalsäurediester mit einer wässrigen Base versetzt, aus dem erhaltenen Gemisch Wasser abdampft, die erhaltene flüssige Phase unter Bildung einer Wasser-in-ÖI-Emulsion mit Wasser versetzt, aus der Emulsion Wasser abdestilliert und den Terephthalsäurediester filtriert.

13. Verfahren nach einem der vorhergehenden Ansprüche, wobei man die Reaktionssuspension erwärmt, indem man sie über einen Wärmetauscher umpumpt.

## Claims

1. A process for preparing a terephthalic diester by reacting terephthalic acid with at least one alcohol, wherein
a) terephthalic acid is suspended in the alcohol in a dispersing tank to obtain a preliminary suspension,
b) the preliminary suspension is passed from the dispersing tank into a reactor and converted in the presence of an esterification catalyst, and
c) water of reaction is distilled off together with the vapor as alcohol-water azeotrope, the vapor is at least partly condensed, the condensate is separated into an aqueous phase and an organic phase, the organic phase is dewatered and the dewatered organic phase is passed at least partly into the dispersing tank,
and the alcohol is selected from linear, branched and cyclic C₈ to C₁₄ aliphatic monools and aromatic monools.

2. The process according to claim 1, wherein the dewatered organic phase has a water content of less than 1000 ppm.

3. The process according to claim 1 or 2, wherein the organic phase is dewatered by stripping with alcohol vapor.

4. The process according to claim 3, wherein the vapor is run counter to the organic phase in a rectifying column in order to obtain an alcohol-enriched organic phase, and the alcohol-enriched organic phase is stripped with alcohol vapor in a stripping column in order to obtain the dewatered organic phase.

5. The process according to any of the preceding claims, wherein the dewatered organic phase is stored in an insulated and/or heated vessel before being introduced into the dispersing tank.

6. The process according to any of the preceding claims, wherein the esterification catalyst is selected from Lewis acids, mineral acids, sulfonic acids and ionic fluids.

7. The process according to claim 6, wherein the esterification catalyst is selected from alkoxides, carboxylates and chelate compounds of titanium, zirconium, tin, hafnium, aluminum and zinc; boron trifluoride, boron trifluoride etherates; sulfuric acid, phosphoric acid; methanesulfonic acid and toluenesulfonic acid.

8. The process according to claims 1 to 7, wherein the esterification catalyst is selected from acidic ion exchangers, zeolites, oxides and/or hydroxides of magnesium, aluminum, zinc, titanium, silicon, tin, lead, antimony, bismuth, molybdenum and manganese.

9. The process according to any of the preceding claims, wherein the esterification catalyst is soluble in the alcohol.

10. The process according to any of the preceding claims, wherein the alcohol is used in such a stoichiometric excess that the crude esterification product comprises 15% to 35% by weight of alcohol.

11. The process according to any of the preceding claims, wherein an inert gas is metered into the reactor and/or a stream of the reaction suspension for fluidization.

12. The process according to any of the preceding claims, wherein the crude terephthalic diester is worked up by admixing with an aqueous base, evaporating water out of the mixture obtained, admixing the liquid phase obtained with water to form a water-in-oil emulsion, distilling water out of the emulsion and filtering the terephthalic diester.

13. The process according to any of the preceding claims, wherein the reaction suspension is heated by pumping it in circulation through a heat exchanger.

## Revendications

1. Procédé de fabrication d'un diester de l'acide téréphtalique par mise en réaction d'acide téréphtalique avec au moins un alcool, selon lequel
a) de l'acide téréphtalique est suspendu dans l'alcool dans une cuve de dispersion, une pré-suspension étant obtenue,
b) la pré-suspension est acheminée depuis la cuve de dispersion dans un réacteur et mise en réaction en présence d'un catalyseur d'estérification, et
c) l'eau de réaction est éliminée par distillation sous la forme d'un azéotrope alcool-eau avec les vapeurs, les vapeurs sont au moins partiellement condensées, le condensat est séparé en une phase aqueuse et une phase organique, la phase organique est déshydratée et la phase organique déshydratée est au moins partiellement acheminée dans la cuve de dispersion,
et l'alcool est choisi parmi les mono-alcools aliphatiques en C₈ à C₁₄ linéaires, ramifiés ou cycliques ou les mono-alcools aromatiques.

2. Procédé selon la revendication 1, dans lequel la phase organique déshydratée présente une teneur en eau inférieure à 1 000 ppm.

3. Procédé selon la revendication 1 ou 2, dans lequel la phase organique est déshydratée par extraction avec une vapeur d'alcool.

4. Procédé selon la revendication 3, dans lequel la phase organique est mise en circulation à contre-courant des vapeurs dans une colonne d'enrichissement afin d'obtenir une phase organique enrichie en alcool, et la phase organique enrichie en alcool est extraite dans une colonne de rectification avec une vapeur d'alcool afin d'obtenir la phase organique déshydratée.

5. Procédé selon l'une quelconque des revendications précédentes, dans lequel la phase organique déshydratée est stockée dans un contenant isolé et/ou chauffé avant l'introduction dans la cuve de dispersion.

6. Procédé selon l'une quelconque des revendications précédentes, dans lequel le catalyseur d'estérification est choisi parmi les acides de Lewis, les acides minéraux, les acides sulfoniques et les fluides ioniques.

7. Procédé selon la revendication 6, dans lequel le catalyseur d'estérification est choisi parmi les alcoolates, les carboxylates et les composés chélatés de titane, de zirconium, d'étain, d'hafnium, d'aluminium et de zinc ; le trifluorure de bore, les éthérates de trifluorure de bore ; l'acide sulfurique, l'acide phosphorique ; l'acide méthanesulfonique et l'acide toluènesulfonique.

8. Procédé selon les revendications 1 à 7, dans lequel le catalyseur d'estérification est choisi parmi les échangeurs d'ions acides, les zéolithes, les oxydes et/ou hydroxydes de magnésium, d'aluminium, de zinc, de titane, de silicium, d'étain, de plomb, d'antimoine, de bismuth, de molybdène et de manganèse.

9. Procédé selon l'une quelconque des revendications précédentes, dans lequel le catalyseur d'estérification est soluble dans l'alcool.

10. Procédé selon l'une quelconque des revendications précédentes, dans lequel l'alcool est utilisé en un excès stoechiométrique tel que le produit d'estérification brut contient 15 à 35 % en poids d'alcool.

11. Procédé selon l'une quelconque des revendications précédentes, dans lequel un gaz inerte est introduit dans le réacteur et/ou un courant de la suspension de réaction pour la fluidisation.

12. Procédé selon l'une quelconque des revendications précédentes, dans lequel, pour le traitement, le diester de l'acide téréphtalique brut est mélangé avec une base aqueuse, l'eau est évaporée du mélange obtenu, la phase liquide obtenue est mélangée avec de l'eau pour obtenir une émulsion eau dans huile, l'eau est éliminée par distillation de l'émulsion et le diester de l'acide téréphtalique est filtré.

13. Procédé selon l'une quelconque des revendications précédentes, dans lequel la suspension de réaction est chauffée par pompage dans un échangeur de chaleur.
